(19) Europäisches Patentamt

European Patent Office

Office européen des brevets

(11) **EP 4 269 552 A1**

(12) **EUROPEAN PATENT APPLICATION**
published in accordance with Art. 153(4) EPC

(43) Date of publication:
**01.11.2023 Bulletin 2023/44**

(21) Application number: **22746046.6**

(22) Date of filing: **28.01.2022**

(51) International Patent Classification (IPC):
**C12M 1/00** $^{(2006.01)}$        **G01N 37/00** $^{(2006.01)}$

(52) Cooperative Patent Classification (CPC):
**C12M 1/00; G01N 37/00**

(86) International application number:
**PCT/JP2022/003394**

(87) International publication number:
**WO 2022/163827 (04.08.2022 Gazette 2022/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.01.2021 JP 2021013761**

(71) Applicant: **FUJIFILM Corporation
Tokyo 106-8620 (JP)**

(72) Inventors:
• **MOCHIZUKI, Aya
Ashigarakami-gun, Kanagawa 258-8577 (JP)**
• **YUKAWA, Hiroyuki
Ashigarakami-gun, Kanagawa 258-8577 (JP)**
• **YASUDA, Kenichi
Ashigarakami-gun, Kanagawa 258-8577 (JP)**
• **OBA, Takahiro
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54) **FLOW PATH DEVICE AND METHOD FOR MANUFACTURING SAME**

(57)    The present disclosure provides a channel device (100, 101, 102, 103, 200, 300, 500, 600, 700) including a flow channel (10) formed of a groove (31) provided in a substrate (30) and a porous membrane (50) having pores (56) communicating in a direction intersecting a thickness direction thereof, in which at least a portion of the porous membrane (50) in contact with the substrate (30) is liquid-tightly sealed along the flow channel (10), and a manufacturing method thereof.

FIG. 1

## Description

## BACKGROUND OF THE INVENTION

### 1. Field of the Invention

The present disclosure relates to a channel device and a manufacturing method thereof.

### 2. Description of the Related Art

A channel device comprises a micrometer-order thin flow channel, and can be used, for example, for cell culture and analysis performed using a small amount of a sample. Therefore, the channel device is widely used in many applications. As such a channel device, a channel device in which a porous membrane is used for a portion of a flow channel is known. For example, JP2016-000051A discloses an organ mimicking device, and JP2017-136082A discloses a cell culture system.

## SUMMARY OF THE INVENTION

Techniques related to a channel device using a porous membrane have been hitherto studied, including techniques of JP2016-000051A, JP2017-136082A, and the like, but liquid leakage due to the use of a porous membrane can be a problem.

The present disclosure has been made in view of such circumstances, and an object to be solved by an embodiment of the present disclosure is to provide a channel device capable of suppressing liquid leakage due to communication of pores in an in-plane direction of a porous membrane.

An object to be solved by another embodiment of the present disclosure is to provide a manufacturing method of the channel device.

The present disclosure includes the following aspects.

<1> A channel device comprising: a flow channel formed of a groove provided in a substrate and a porous membrane having pores communicating in a direction intersecting a thickness direction of the porous membrane,
in which at least a portion of the porous membrane in contact with the substrate is liquid-tightly sealed along the flow channel.
<2> The channel device according to <1>, further comprising: a plurality of the flow channels.
<3> The channel device according to <2>, in which the channel device includes two flow channels facing each other through the porous membrane.
<4> The channel device according to any one of <1> to <3>, further comprising: a variable pressure chamber that is positioned adjacent to the flow channel in a plane direction of the porous membrane and extends along at least a portion of the flow channel.

<5> The channel device according to <4>, further comprising: the two variable pressure chambers positioned on both sides of the flow channel.
<6> The channel device according to any one of <1> to <5>, in which at least the portion of the porous membrane in contact with the substrate is filled with a polymer material.
<7> The channel device according to any one of <1> to <5>, in which at least the portion of the porous membrane in contact with the substrate is compressed.
<8> The channel device according to any one of <1> to <5>, in which at least the portion of the porous membrane in contact with the substrate is melted.
<9> The channel device according to any one of <1> to <8>, in which the porous membrane has an opening ratio of 10% to 70% and an average opening diameter of 0.01 $\mu$m to 100 $\mu$m.
<10> The channel device according to any one of <1> to <9>, in which a layer of an extracellular matrix is formed on a surface of a portion of the porous membrane that forms the flow channel.
<11> The channel device according to <10>, in which the extracellular matrix is not provided in the groove.
<12> A manufacturing method of a channel device, comprising: a step of disposing a porous membrane having pores communicating in a direction intersecting a thickness direction of the porous membrane on a first substrate having a first groove to form a first flow channel formed of the first groove and the porous membrane, and liquid-tightly sealing at least a portion of the porous membrane in contact with the first substrate along the first flow channel.
<13> The manufacturing method of a channel device according to <12>, in which the sealing is performed by filling at least the portion of the porous membrane in contact with the first substrate with a polymer material.
<14> The manufacturing method of a channel device according to <12>, in which the sealing is performed by compressing at least the portion of the porous membrane in contact with the first substrate.
<15> The manufacturing method of a channel device according to <12>, in which the sealing is performed by melting at least the portion of the porous membrane in contact with the first substrate.
<16> The manufacturing method of a channel device according to any one of <12> to <15>, in which the first substrate has two first side grooves positioned on both sides of the first groove and provided along at least a portion of the first groove, the manufacturing method further comprising:
a step of disposing a second substrate having a second groove and two second side grooves on the first substrate to cause the second groove and the two second side grooves to respectively face the first groove and the two first side grooves through the porous membrane to form a second flow channel formed of the second groove and the porous membrane and two variable pressure chambers formed of the first side grooves and the second side grooves.

According to one embodiment of the present disclosure, there is provided a channel device in which liquid leakage due to communication of pores in an in-plane direction of a porous membrane is suppressed.

According to another embodiment of the present disclosure, there is provided a manufacturing method of the channel device.

**BRIEF DESCRIPTION OF THE DRAWINGS**

Fig. 1 is a schematic plan view showing an example of a channel device.
Fig. 2 is a schematic cross-sectional view of the channel device of Fig. 1 taken along the line A-A.
Fig. 3 is a schematic plan view showing an example of a porous membrane.
Fig. 4 is a schematic cross-sectional view showing an example of the channel device.
Fig. 5 is a schematic cross-sectional view showing an example of the channel device.
Fig. 6 is a schematic cross-sectional view showing an example of the channel device.
Fig. 7 is a schematic cross-sectional view showing an example of the channel device.
Fig. 8 is a schematic plan view showing an example of the channel device.
Fig. 9 is a schematic cross-sectional view of the channel device of Fig. 10 taken along the line B-B.
Fig. 10 is a schematic cross-sectional view showing an example of a channel device.
Fig. 11 is a schematic cross-sectional view showing an example of the channel device.
Fig. 12 is a schematic cross-sectional view showing an example of the channel device.
Fig. 13 is a schematic cross-sectional view showing an example of the channel device.
Fig. 14 is a schematic cross-sectional view showing an example of the channel device.
Fig. 15 is a schematic cross-sectional view showing an example of a first substrate.
Fig. 16 is a schematic cross-sectional view showing an example of a form in which a second substrate is disposed on the first substrate of Fig. 15 through the porous membrane.
Fig. 17 is a schematic cross-sectional view showing an example of the form in which the second substrate is disposed on the first substrate of Fig. 15 through the porous membrane.
Fig. 18 is a schematic cross-sectional view showing an example of the form in which the second substrate is disposed on the first substrate of Fig. 15 through the porous membrane.
Fig. 19 is a schematic cross-sectional view showing an example of the form in which the second substrate is disposed on the first substrate of Fig. 15 through the porous membrane.
Fig. 20 is a schematic cross-sectional view showing an example of the form in which the second substrate is disposed on the first substrate of Fig. 15 through the porous membrane.
Fig. 21 is a schematic cross-sectional view showing an example of the first substrate.
Fig. 22 is a schematic cross-sectional view showing an example of a form in which the second substrate is disposed on the first substrate of Fig. 21 through the porous membrane.
Fig. 23 is a schematic cross-sectional view showing an example of the form in which the second substrate is disposed on the first substrate of Fig. 21 through the porous membrane.
Fig. 24 is a schematic cross-sectional view showing an example of the form in which the second substrate is disposed on the first substrate of Fig. 21 through the porous membrane.
Fig. 25 is a schematic cross-sectional view showing an example of the form in which the second substrate is disposed on the first substrate of Fig. 21 through the porous membrane.
Fig. 26 is a schematic plan view showing a first substrate of Examples.
Fig. 27 is a schematic plan view showing a second substrate of Examples.

**DESCRIPTION OF THE PREFERRED EMBODIMENTS**

Hereinafter, details of a channel device and a manufacturing method thereof according to an embodiment of the present disclosure will be described.
In the present disclosure, a numerical range indicated by using "to" means a range including numerical values before and after "to" as a minimum value and a maximum value, respectively.
In a numerical range described stepwise in the present disclosure, an upper limit or a lower limit a described in a certain numerical range may be replaced with an upper limit or a lower limit of another numerical range described stepwise. In addition, in a numerical range described in the present disclosure, an upper limit or a lower limit described in a certain numerical range may be replaced with a value shown in Examples.
In the present disclosure, a combination of two or more preferred aspects is a more preferred aspect.
In the present disclosure, the amount of each component means the total amount of a plurality of kinds of substances in a case where a plurality of kinds of substances corresponding to each component are present, unless otherwise specified.
In the present disclosure, the term "step" is included not only in an independent step but also in this term as long as an intended purpose of the step is achieved, even in a case where the step cannot be clearly distinguished from other steps.

In the present disclosure, "(meth)acrylic" is a term used in a concept including both acrylic and methacrylic, and "(meth)acrylate" is a term used in a concept including both acrylate and methacrylate.

In the present disclosure, the term "total solid content amount" refers to the total mass of components excluding a solvent from the total composition. In addition, the term "solid content" is a component obtained by removing a solvent from the total composition, and may be a solid or a liquid, for example, at 25°C.

The drawings referred to in the following description are illustrative and schematically shown, and the present disclosure is not limited to these drawings. Like reference numerals denote like elements. In addition, reference numerals in the drawings may be sometimes omitted.

<Channel Device>

The channel device according to the embodiment of the present disclosure has a flow channel formed of a groove provided in a substrate and a porous membrane having pores communicating in a direction intersecting a thickness direction thereof, in which at least a portion of the porous membrane in contact with the substrate is liquid-tightly sealed along the flow channel.

As the channel device, a channel device in which a porous membrane is used as a portion of a flow channel is known. In order to efficiently perform cell culture, analysis, and the like, as the porous membrane, a porous membrane in which pores communicate not only in a thickness direction but also in an in-plane direction, that is, in a direction intersecting the thickness direction is suitably used. However, in a case of using the porous membrane in which the pores communicate not only in the thickness direction but also in the in-plane direction, liquid leakage due to the communication of the pores in the in-plane direction becomes a problem.

For example, JP2016-000051A and JP2017-136082A disclose a channel device having a structure in which both sides of a porous membrane are sandwiched between two substrates in a thickness direction. However, in the channel device of JP2016-000051A and JP2017-136082A, since the porous membrane in which pores communicate only in the thickness direction is used, in a case where a porous membrane in which pores communicate also in an in-plane direction is used, liquid leakage due to the communication of the pores in the in-plane direction occurs.

Contrary to this, in the channel device according to the embodiment of the present disclosure, at least the portion of the porous membrane in contact with the substrate is liquid-tightly sealed along the flow channel. Therefore, it is possible to suppress liquid leakage due to the communication of the pores in the in-plane direction of the porous membrane.

[Flow Channel]

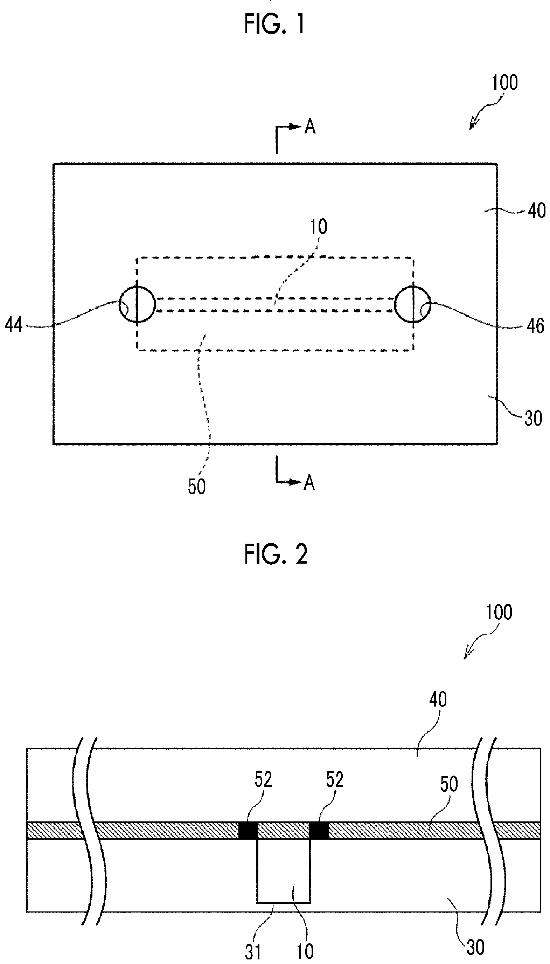

The flow channel is formed of the groove provided in the substrate and the porous membrane in which the pores communicate in the direction intersecting the thickness direction (hereinafter, sometimes simply referred to as a "porous membrane"). For example, in a channel device 100 shown in Figs. 1 and 2, a porous membrane 50 is disposed on a first substrate 30 having a groove 31 to form a flow channel 10. In addition, at least a portion 52 of the porous membrane 50 in contact with the first substrate 30 is liquid-tightly sealed along the flow channel 10. Accordingly, it is possible to suppress liquid leakage due to communication of pores of the porous membrane 50 in an in-plane direction. At least the portion of the porous membrane that is liquid-tightly sealed is sometimes referred to as a "sealing portion".

In the present disclosure, the term "liquid-tight" means that there is no liquid leakage due to the communication of the pores of the porous membrane in the in-plane direction after one day has passed in a state where a liquid has flowed into the flow channel.

For example, in the channel device 100 shown in Figs. 1 and 2 (cross-sectional view of the channel device 100 in Fig. 1 taken along the line A-A), a second substrate 40 facing the first substrate 30 is disposed on the first substrate 30 through the porous membrane 50. As shown in Fig. 1, the second substrate 40 has an inlet 44 and an outlet 46 communicating with the flow channel 10, and a sample is allowed to flow in the flow channel 10 through the inlet 44 and the outlet 46. The inlet and the outlet may be reversed.

A width and a height of the flow channel (for example, in Fig. 2, a length in the in-plane direction and a length in the thickness direction of the first substrate 30, respectively) are not particularly limited and are usually about 50 $\mu$m to 2,000 $\mu$m.

[Substrate]

A material of the substrate is not particularly limited, but is preferably made of a resin from the viewpoint of easy manufacturing. In addition, in a case where the channel device includes a variable pressure chamber described later, the substrate is preferably made of a resin from the viewpoint of facilitating deformation of a flow channel wall due to pressurization and depressurization. In a case where the substrate is made of a resin, examples of the material of the substrate include polydimethylsiloxane, an acrylic resin, a urethane resin, a urethane-acrylic resin, an epoxy resin, polyethylene terephthalate (PET), polyethylene naphthalate (PEN), polycarbonate (PC), an acrylic-polycarbonate resin, triacetylcellulose (TAC), a polyolefin, a cyclo olefin polymer (COP), and an acrylonitrile-butadiene-styrene copolymer resin (ABS resin). From the viewpoint of deformability, the material of the substrate is preferably polydimethylsiloxane. As the polydimethylsiloxane, a commercially available product can be used, and examples thereof include "SYLGARD 184" (a two-part type consisting of a base agent and a curing agent) manufactured by Dow.

A thickness of the substrate is not particularly limited, but is usually about 100 $\mu$m to 10 mm.

[Porous Membrane]

In the porous membrane, the pores communicate in the direction intersecting the thickness direction. In addition, it is preferable that the porous membrane has such a structure and thus has a high opening ratio.

The porous membrane can reproduce a tissue interface close to that of a living body, for example, in cell culture applications. Therefore, by using the channel device according to the embodiment of the present disclosure, it is possible to evaluate an interaction between cells with higher accuracy. In cell culture applications, the porous membrane functions as a scaffold to which cells adhere. In addition, as will be described later in detail, the porous membrane can be suitably used as a substrate for cell culture by forming a layer of an extracellular matrix on a surface of the porous membrane.

In the present disclosure, the "pore" of the porous membrane means a space existing in the porous membrane and being partitioned from each other by a side wall. However, the adjacent pores communicate with each other.

Hereinafter, an example of the porous membrane will be described with reference to the drawings. In the following description, the term "major axis" means a maximum length of a distance between any two points on a contour.

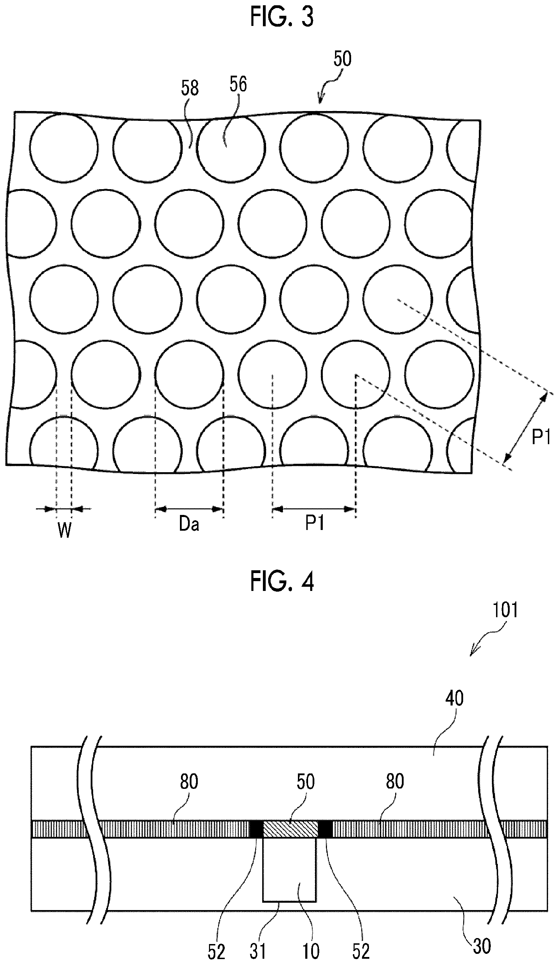

Fig. 3 is a schematic plan view of an exemplary porous membrane 50 as viewed from an upper surface side. In the porous membrane 50, pores 56 are disposed over an entire principal surface. However, for example, in cell culture applications, in a case where the porous membrane 50 has a region that the cells cannot come into contact with, the pores 56 may not be disposed in the region that the cells cannot come into contact with.

In the thickness direction of the porous membrane, the pores 56 may be through-holes or non-through-holes. For example, in a case of performing double-sided culture in which cells of the same type or different types are cultured on both sides of the porous membrane in cell culture applications, from the viewpoint of promoting cell-cell interaction on both sides of the porous membrane, the pores of the porous membrane are preferably through-holes in the thickness direction of the porous membrane. In addition, from the viewpoint of further improving the deformability of the porous membrane, the pores 56 are preferably through-holes in the thickness direction of the porous membrane.

The porous membrane 50 shown in Fig. 3 is an example having a honeycomb structure. The honeycomb structure refers to a structure in which pores are arranged in a honeycomb pattern. The honeycomb-like arrangement is an arrangement in which hexagonal parallelogons (preferably regular hexagons) or similar shapes are used as units, and centroids of openings are positioned at vertices and intersections of diagonals of these figures. The term "centroid" means a centroid of a two-dimensional figure of the opening on the principal surface. Since the porous membrane 50 has the honeycomb structure, the opening ratio can be increased, and better deformability can be obtained. In addition, for example, in a case where cells are subjected to double-sided culture using the porous membrane 50 in cell culture applications, it is preferable to increase the opening ratio because the interaction between the cells on each surface is efficiently performed.

The arrangement of the pores of the porous membrane 50 is not limited to the honeycomb structure, and the porous membrane 50 may have a lattice arrangement, a face-centered lattice arrangement, or the like.

The lattice arrangement is an arrangement in which parallelograms (needless to say, squares, rectangles, and rhombuses are included, preferably squares) or similar shapes are used as units, and centroids of openings are positioned at vertices of these figures.

The face-centered lattice arrangement is an arrangement in which parallelograms (needless to say, squares, rectangles, and rhombuses are included, preferably squares) or similar shapes are used as units, and centroids of openings are positioned at vertices and intersections of diagonals of these figures.

For example, in cell culture applications, from the viewpoint of improving homogeneity of cell layers formed on the porous membrane, it is preferable that the pores 56 in the porous membrane 50 are regularly arranged. As a standard for regular arrangement is an arrangement in which a coefficient of variation of areas of the hexagonal parallelogons or the parallelograms, which are the units of the arrangement, is 10% or less. The coefficient of variation is obtained for any 10 units of the arrangement.

A shape of the pore 56 is not particularly limited. Examples of the shape of the pore 56 include a spherical segment shape lacking a part of a sphere, a barrel shape, a cylindrical shape, and a prismatic shape.

Examples of a shape of the opening of the pore 56 include a circular shape, an elliptical shape, and a polygonal shape. The opening of the porous membrane 50 means an inlet portion of the pore 56 formed in at least one of two principal surfaces of the porous membrane 50.

The opening ratio of the porous membrane is preferably 10% to 70%. By setting the opening ratio of the porous membrane to 10% or more, a porous membrane having better deformability can be obtained. In addition, by setting the opening ratio of the porous membrane to 70% or less, a porous membrane having a better self-supporting property can be obtained. The opening ratio of the porous membrane is more preferably 20% to 70% and even more preferably 30% to 60%. In the present disclosure, the opening ratio of the porous membrane means a ratio of opening areas to a unit surface area. In a case where the openings are present on both surfaces of the porous membrane, it is preferable that the opening ratio in at least one surface is 30% to 60%. The opening ratio can be obtained by the following method.

The surface of the porous membrane is imaged with a microscope, and areas (S1) of the openings are calculated from the number of pixels imaged. Similarly, an area (S2) of the porous membrane including the openings is calculated. The opening ratio (P) is obtained by the following formula.

$$P = S1 / S2 \times 100$$

A pitch P1 between the pores 56 is a distance between centers of the openings adjacent to each other. For example, in cell culture applications, the pitch P1 is preferably set according to a size of the cell to be cultured on the porous membrane 50. The pitch P1 may be, for example, 0.01 $\mu$m to 100 $\mu$m.

An opening diameter Da is a major axis of the opening of the pore 56 on the surface of the porous membrane 50. An average opening diameter, which is an average value of the opening diameters Da, may be, for example, 0.1% to 200% with respect to the major axis (for example, 10 $\mu$m to 50 $\mu$m) of the cell to be seeded. The average opening diameter can be appropriately set depending on an intended purpose. From the viewpoint of increasing the opening ratio of the porous membrane 50, the average opening diameter is preferably 0.01 $\mu$m or more, more preferably 0.1 $\mu$m or more, even more preferably 0.5 $\mu$m or more, and particularly preferably 1 $\mu$m or more. From the viewpoint of a scaffold for cell adhesion, the average opening diameter is preferably 100 $\mu$m or less, more preferably 50 $\mu$m or less, even more preferably 20 $\mu$m or less, and particularly preferably 10 $\mu$m or less. From the above viewpoints, the average opening diameter is preferably 0.01 $\mu$m to 100 $\mu$m, more preferably 0.1 $\mu$m to 50 $\mu$m, even more preferably 0.5 $\mu$m to 20 $\mu$m, and particularly preferably 1 $\mu$m to 10 $\mu$m.

The average opening diameter is determined by the following method. Any 300 $\mu$m square range of the surface of the porous membrane is imaged with a microscope, and an area of each of all the pores within the range is calculated from the number of pixels imaged. A diameter of a circle corresponding to the pore area is defined as the opening diameter, and an arithmetic average value thereof is defined as the average opening diameter.

From the viewpoint of deformability, the self-supporting property, and strength, the porous membrane preferably has an opening ratio of 10% to 70% and an average opening diameter of 1 $\mu$m to 30 $\mu$m.

A coefficient of variation of the opening diameters Da is preferably 20% or less, and is preferably as small as possible. For example, in cell culture applications, as the coefficient of variation of the opening diameters Da decreases, the homogeneity of the cell layers formed on the porous membrane 50 tends to increase. The coefficient of variation of the opening diameters Da is obtained for any 10 pores.

A width W of a side wall 58 is a length of a width of the side wall 58 on a line segment connecting the centers of the openings adjacent to each other. From the viewpoint of maintaining the self-supporting property of the porous membrane and improving handleability, the width W is preferably 0.5 $\mu$m or more, more preferably 1 $\mu$m or more, and even more preferably 3 $\mu$m or more.

From the viewpoint of producing a channel device, for example, a thickness of the porous membrane is preferably 0.01 $\mu$m to 100 $\mu$m, more preferably 0.1 $\mu$m to 50 $\mu$m, and even more preferably 1 $\mu$m to 20 $\mu$m.

Although the porous membrane 50 shown in Fig. 3 is a single-layer membrane, the porous membrane 50 may also be a laminated membrane in which a plurality of porous membranes are laminated.

A material of the porous membrane is not particularly limited. Examples of the material of the porous membrane include polymers such as polydimethylsiloxane, polybutadiene, polystyrene, polycarbonate, polyester (for example, polylactic acid, polycaprolactone, polyglycolic acid, a polylactic acid-polyglycolic acid copolymer, a polylactic acid-polycaprolactone copolymer, polyethylene terephthalate, polyethylene naphthalate, polyethylene succinate, polybutylene succinate, and poly-3-hydroxybutyrate), polyacrylate, polymethacrylate, polyacrylamide, polymethacrylamide, polyvinyl chloride, polyvinylidene chloride, polyvinylidene fluoride, polyhexafluoropropene, polyvinyl ether, polyvinylcarbazole, polyvinyl acetate, polytetrafluoroethylene, polylactone, polyamide, polyimide, polyurethane, polyurea, polyaromatics, polysulfone, polyethersulfone, a polysiloxane derivative, and a cellulose acylate (for example, triacetylcellulose, cellulose acetate propionate, and cellulose acetate butyrate). As necessary, the polymer may be a homopolymer, a copolymer, a polymer blend, or a polymer alloy, from the viewpoint of solubility in a solvent, optical properties, electrical properties, membrane strength, elasticity, and the like. One type of the polymer may be used alone, or two or more types thereof may be used in combination.

From the viewpoint of the self-supporting property, the material of the porous membrane is preferably at least one selected from the group consisting of polybutadiene, polyurethane, polystyrene, and polycarbonate. For example, in cell culture applications, from the viewpoint of facilitating maintenance of engraftment of the cell layers, the material of the porous membrane is preferably at least one selected from the group consisting of a polylactic acid, a polylactic acid-polyglycolic acid copolymer, and a polylactic acid-polycaprolactone copolymer. From the viewpoint of achieving better deformability, an elastomer such as polybutadiene or polyurethane is preferable.

-Manufacturing Method of Porous Membrane-

The manufacturing method of the porous membrane is not particularly limited. Examples of the manufacturing method of the porous membrane include: a manufacturing method in which a porous membrane is manufactured by performing etching, blasting, or punching on a membrane made of a resin and forming throughholes in the membrane; and a manufacturing method in which water droplets are grown on a coating film containing a polymer and a solvent to form through-holes, as described in JP4734157B, JP4904025B, JP4945281B, JP5405374B, JP5422230B, and JP2011-74140A.

-Extracellular Matrix-

In cell culture applications, it is preferable that a layer of an extracellular matrix is formed on the surface of the portion of the porous membrane that forms the flow channel. Such a porous membrane can be suitably used as a substrate for cell culture. The extracellular matrix is a biopolymer present outside the cell. The extracellular matrix functions as a scaffold for cell culture and can also exert an effect on proliferation, differentiation, and gene expression of cells. By forming the layer of the extracellular matrix on the surface of the porous membrane, a wide cell adhesion surface is secured, and a desired effect of the extracellular matrix can be suitably obtained. The extracellular matrix may be contained in the pores of the porous membrane.

Examples of the extracellular matrix include at least one selected from the group consisting of fibronectin, collagen (for example, Type I collagen, Type IV collagen, or Type V collagen), laminin, vitronectin, gelatin, perlecan, nidogen, proteoglycan, osteopontin, tenascin, nephronectin, a basement membrane matrix, and polylysine. As the basement membrane matrix, commercially available products (for example, MATRIGEL (registered trademark) and Geltrex (registered trademark)) are available.

A thickness of the layer of the extracellular matrix is not particularly limited, and may be, for example, 0.01% to 30%, 0.01% to 20%, or 0.01% to 10% with respect to the thickness of the porous membrane.

-Manufacturing Method of Substrate for Cell Culture-

A manufacturing method of a substrate for cell culture is not particularly limited. For example, the extracellular matrix layer can be formed on the surface of the porous membrane by deploying an extracellular matrix-containing solution on a plate such as a glass plate to be transferred onto the surface of the porous membrane.

A concentration of the extracellular matrix-containing solution can be appropriately adjusted. As an example, in a case where the extracellular matrix is collagen, the concentration of the collagen solution may be 0.3 mg/mL to 10 mg/mL, 1.0 mg/mL to 10 mg/mL, or 4.0 mg/mL to 10 mg/mL.

In addition, as a method of forming the layer of the extracellular matrix on the surface of the porous membrane, there is a method in which a channel device is manufactured using a porous membrane before forming a layer of an extracellular matrix, and an extracellular matrix-containing solution is allowed to flow in a flow channel of the channel device. However, in this method, the consumption of the extracellular matrix-containing solution is large, and it is difficult to uniformly close the pores of the surface of the porous membrane. In addition, the extracellular matrix contained in the extracellular matrix-containing solution adheres to the groove of the flow channel.

Therefore, in a case where an extracellular matrix is used, it is preferable that a substrate for cell culture is produced by forming a layer of the extracellular matrix on a surface of a porous membrane in advance and a channel device is manufactured using the substrate for cell culture. The channel device thus obtained has the porous membrane in which pores are uniformly closed by the layer of the extracellular matrix, and a groove of a flow channel does not have the extracellular matrix.

-Properties of Substrate for Cell Culture-

(Thickness)

From the viewpoint of producing the channel device, a thickness of the substrate for cell culture is preferably 0.01 $\mu$m to 100 $\mu$m, more preferably 0.1 $\mu$m to 50 $\mu$m, and even more preferably 1 $\mu$m to 20 $\mu$m.

For example, a planar extracellular matrix membrane that does not use a porous membrane cannot maintain a self-supporting property and is inferior in handleability in a case where the planar extracellular matrix membrane has a small thickness. However, the substrate for cell culture of the embodiment of the present disclosure can maintain a self-supporting property even in a case where the substrate for cell culture of the embodiment of the present disclosure has a thickness of, for example, 40 $\mu$m or less, preferably 20 $\mu$m or less, and more preferably 8 $\mu$m or less. Therefore, the substrate for cell culture of the embodiment of the present disclosure is useful in that both the deformability and the self-supporting property can be achieved even if the thickness is small.

The thickness of the substrate for cell culture can be measured by microscopic observation.

(Young's modulus)

A Young's modulus of the substrate for cell culture obtained by a tensile test based on JIS K 7161-1:2014 and JIS K 7127:1999 is preferably 2.0 MPa or less, more preferably 1.5 MPa or less, and even more preferably 1.2 MPa or less. A Young's modulus of 2.0 MPa or less indicates that the substrate for cell culture is excellent in deformability. A lower limit of the Young's modulus is not particularly limited and is preferably 0.1 MPa or more from the viewpoint of strength of the substrate for cell culture. Specifically, the Young's modulus can be obtained by the method described in Examples.

(Maximum Elongation Rate)

A maximum elongation rate of the substrate for cell culture obtained by the tensile test based on JIS K 7161-1 and JIS K 7127:1999 is preferably 130% or more, more preferably 140% or more, and even more preferably 150% or more. A maximum elongation rate of 130% or more, preferably 140% or more, and more preferably 150% or more indicates that the substrate for cell culture is not easily torn even in a case where the substrate for cell culture is elongated. An upper limit of the maximum elongation rate is not particularly limited, and from the viewpoint of handleability of the substrate for cell culture, the maximum elongation rate may be 500% or less. Specifically, the maximum elongation rate can be obtained by the method described in Examples.

The substrate for cell culture can be widely used for production of a material for transplantation into a living body, a tissue model for drug evaluation or pathological condition evaluation, a test tissue as an alternative to an animal experiment, and the like. In particular, the substrate for cell culture can be suitably used for applications in which mechanical stimulation to cells during culture or evaluation is useful. In addition, according to the channel device according to the embodiment of the present disclosure, culture similar to planar culture is possible, and events such as cells passing through the pores of the porous membrane and falling off can be suppressed. Therefore, the channel device according to the embodiment of the present disclosure is suitable for production of a tissue with a few defects such as holes.

A type of the cell to be cultured is not particularly limited. For example, the cell may be a dividing cell or a non-dividing cell. In the present disclosure, "culture" does not necessarily have to be accompanied by proliferation of cells, and is included in this term as long as the viability of cells is maintained regardless of the presence or absence of proliferation.

Examples of the cell to be cultured include at least one selected from the group consisting of a parenchymal cell (for example, a hepatic parenchymal cell or a pancreatic parenchymal cell), a stromal cell (for example, a pericyte), a muscle cell (for example, a smooth muscle cell, a cardiac muscle cell, or a skeletal muscle cell), a fibroblast, a nerve cell, an endothelial cell (for example, a vascular endothelial cell or a lymphatic endothelial cell), an epithelial cell (for example, an alveolar epithelial cell, an oral epithelial cell, a bile duct epithelial cell, an intestinal epithelial cell, a pancreatic duct epithelial cell, a renal epithelial cell, a renal tubular epithelial cell, or a placental epithelial cell), and a cell capable of differentiating into any of the above cells (for example, a progenitor cell, a mesenchymal stem cell, or a pluripotent stem cell).

Examples of the pluripotent stem cell include an embryonic stem cell (ES cell), an induced pluripotent stem cell (iPS cell), an embryonic germ cell (EG cell), an embryonal carcinoma cell (EC cell), a multipotent adult progenitor cell (MAP cell), an adult pluripotent stem cell (APS cell), and a multi-lineage differentiating stress enduring cell (Muse cell). The pluripotent stem cell can be differentiated into a somatic cell by adding a differentiation-inducing factor that induces differentiation into a somatic cell of interest to a culture medium.

As the cell to be cultured, a cell having a genetic mutation or a cell derived from a patient may be used for the purpose of reproducing a pathological condition.

The substrate for cell culture may be used for monoculture of one type of cell or for co-culture of a plurality of types of cells. There are cases where co-culture of a plurality of types of cells instead of simple culture of one type of cell enables growth and proliferation of cells in an environment more similar to a living body via cell-cell interaction and results in an increase in a biomimetic property.

The substrate for cell culture may be used for single-sided culture or double-sided culture. In a case of performing double-sided culture, the types of cells to be cultured on each surface may be the same or different. In particular, in a case where the porous membrane is a porous membrane having through-holes, the cells on each surface during double-sided culture can interact satisfactorily via the extracellular matrix.

In an embodiment, first cells may be cultured on one surface of the substrate for cell culture to form a first cell layer, and second cells different from the first cells may be cultured on the opposite surface to form a second cell layer. More specifically, for example, a vascular endothelial cell layer may be used as the first cells, smooth muscle cells may be used as the second cells, and both types of cells may be subjected to co-culture through the porous membrane to produce a blood vessel-mimicking structure (vascular wall model). According to such a method, it is possible to improve a biomimetic property of the vascular wall model by the interaction between the vascular endothelial cells and the smooth muscle cells. Furthermore, since the substrate for cell culture has good cell adhesion, it is possible to produce a biological membrane having a few defects such as holes.

In the vascular wall model, it is preferable that chemical substances do not freely pass between the cells of the vascular endothelial cell layer, that is, a barrier function is provided. In the vascular wall model that can be produced using the substrate for cell culture of the embodiment of the present disclosure, it is presumed that cell-cell adhesion of the vascular endothelial cells is developed in a state similar to a vascular wall in a living body. In order to perform drug evaluation more accurately using the vascular wall model, it is desirable that the vascular wall model has a structure and a function similar to those of a vascular wall in a living body. The vascular wall model that can be produced using the substrate for cell culture of the embodiment of the present disclosure can be an excellent measure for drug evaluation.

The cells may be suspended in a liquid culture medium to be seeded on the substrate for cell culture as a cell suspension. The liquid culture medium used for the preparation of the cell suspension or the cell culture is selected according to a target cell type. Specific examples of the culture medium include culture media optimized according to cell types by adding a cell proliferation factor to a basal culture medium for mammalian cells, such as Dulbecco's Modified Eagle's Medium (DMEM), Dulbecco's Modified Eagle Medium/Nutrient Mixture F-12 (DMEM:F-12), Eagle's minimal essential medium (EMEM), Minimum Essential Medium Alpha (MEMα), and Basal Medium Eagle (BME). Commercially available products of such a culture medium are available. The liquid culture medium may be a culture medium in which a plurality of types of media are mixed. A pH of the liquid culture medium is, for example, 7.0 to 8.0.

[Sealing Portion]

At least a portion of the porous membrane in contact with the substrate is liquid-tightly sealed along the flow channel to form the sealing portion. An aspect of the sealing portion is not particularly limited as long as the porous membrane is liquid-tightly sealed along the flow channel, and may be appropriately selected in consideration of an application of the channel device, a manufacturing method, and the like. The sealing portion is preferably, as shown below, (1) a sealing portion formed by filling the porous membrane with a polymer material, (2) a sealing portion formed by compressing the porous membrane, or (3) a sealing portion formed by melting the porous membrane.

-Sealing Portion Formed by Filling Porous Membrane with Polymer Material-

In the sealing portion, at least a portion of the porous membrane in contact with the substrate may be filled with the polymer material.

In the present disclosure, in the sealing portion, the fact that the porous membrane is "filled" with the polymer material means that the polymer material is present in the pores of the porous membrane to such an extent that liquid-tightness is obtained, and does not necessarily mean that the entire pores in the porous membrane are filled with the polymer material.

In addition, by confirming that the liquid-tightness of the sealing portion can be obtained, that is, the liquid leakage due to the communication of the pores in the in-plane direction of the porous membrane in the channel device can be suppressed, it can be determined that the porous membrane is filled with the polymer material.

From the viewpoint of further enhancing the liquid-tightness, a higher filling ratio of the polymer material in the sealing portion (a ratio of a volume of the polymer material to an entire volume of the pores in the porous membrane in the sealing portion) is preferable as high as possible, preferably 50 volume% or more, more preferably 80 volume%, and most preferably 100 volume%.

For example, in the channel device 100 shown in Fig. 2 and a channel device 101 shown in Fig. 4, the porous membrane 50 is disposed on the first substrate 30 having the groove 31 to form the flow channel 10, and a sealing portion 52 formed by filling at least a portion of the porous membrane 50 in contact with the first substrate 30 with the polymer material is included. Accordingly, it is possible to suppress the liquid leakage due to the communication of the pores of the porous membrane 50 in an in-plane direction. In addition, the second substrate 40 facing the first substrate 30 is disposed on the first substrate 30 through the porous membrane 50.

The channel device 101 shown in Fig. 4 is an example including the porous membrane 50 having a width closer to that of the flow channel 10 than the channel device 100 shown in Fig. 2. In addition, in the channel device 101 shown in Fig. 4, in addition to the sealing portion 52, a polymer material 80, which is the same polymer material as the sealing portion 52, is filled between the first substrate 30 and the second substrate 40.

The polymer material is not particularly limited, and examples thereof include polydimethylsiloxane, an acrylic resin, a urethane resin, a urethane-acrylic resin, an epoxy resin, polyethylene terephthalate (PET), polyethylene naphthalate (PEN), polycarbonate (PC), an acrylic-polycarbonate resin, triacetylcellulose (TAC), a polyolefin, a cyclo olefin polymer (COP), and an acrylonitrile-butadiene-styrene copolymer resin (ABS resin).

One type of the polymer material may be used alone, or two or more types thereof may be used in combination. In addition, the polymer material can function as an adhesive material for adhesion between the substrate and the porous membrane.

From the viewpoint of enhancing adhesiveness between the substrate and the porous membrane, the polymer material may be an epoxy-based material.

In a case where the substrate is transparent, it is possible to obtain a channel device capable of visually recognizing the flow channel. However, from the viewpoint of improving visibility of the flow channel by reducing a difference in refractive index between the substrate and the polymer material, the polymer material is preferably a material of the same type as the substrate. For example, both the material of the substrate and the polymer material may be an acrylic material, a urethane-based material, or the like.

In the case where the channel device includes the variable pressure chamber described later, the polymer material is preferably a siloxane-based material, for example, polydimethylsiloxane, from the viewpoint of facilitating deformation of a flow channel wall (details will be described later) due to pressurization and depressurization. As the polydimethylsiloxane, a commercially available product can be used, and examples thereof include "SYLGARD 184" (a two-part type consisting of a base agent and a curing agent) manufactured by Dow.

In addition, in considering the deformability, it is preferable to appropriately select the polymer material in consideration of the material of the substrate.

-Sealing Portion Formed by Compressing Porous Membrane-

In the sealing portion, at least a portion of the porous membrane in contact with the substrate may be compressed.

In the present disclosure, the fact that the porous membrane is "compressed" in the sealing portion means that the porous membrane is compressed to such an extent that liquid-tightness is obtained and the pores are blocked.

In addition, by confirming that the liquid-tightness of the sealing portion can be obtained, that is, the liquid leakage due to the communication of the pores in the in-plane direction of the porous membrane in the channel device can be suppressed, it can be determined that the porous membrane is compressed.

An aspect in which the porous membrane is compressed is not particularly limited, and may be bending of the porous membrane, pressing of the porous membrane, or the like.

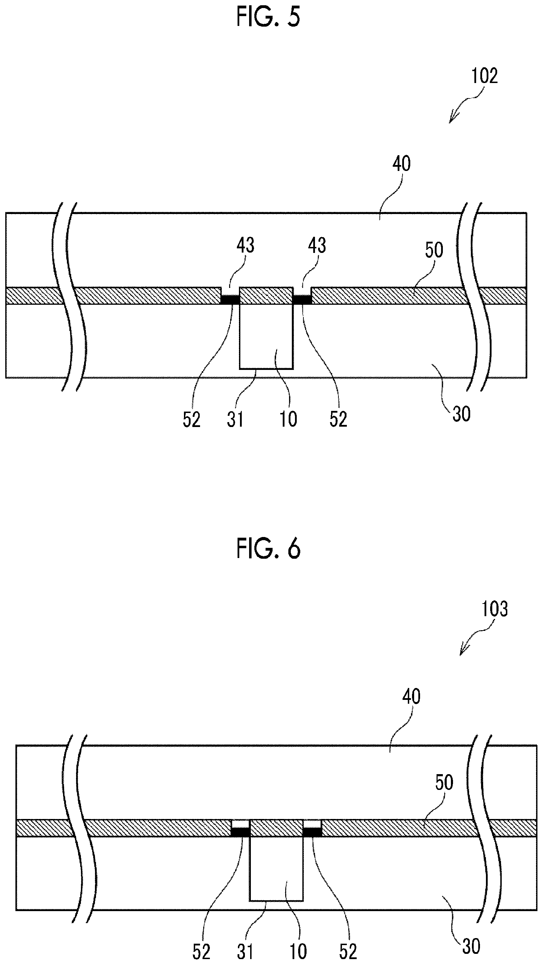

For example, in a channel device 102 shown in Fig. 5, the porous membrane 50 is disposed on the first substrate 30 having the groove 31 to form the flow channel 10, and the sealing portion 52 formed by pressing a protruding portion 43 of the second substrate 40 against at least a portion of the porous membrane 50 in contact with the first substrate 30 is included. Accordingly, it is possible to suppress the liquid leakage due to the communication of the pores of the porous membrane 50 in an in-plane direction. In addition, the second substrate 40 facing the first substrate 30 is disposed on the first substrate 30 through the porous membrane 50.

-Sealing Portion Formed by Melting Porous Membrane-

In the sealing portion, at least a portion of the porous membrane in contact with the substrate may be melted.

In the present disclosure, the fact that the porous membrane is "melted" in the sealing portion means that the porous membrane is melted to such an extent that liquid-tightness is obtained and the pores are blocked.

In addition, by confirming that the liquid-tightness of the sealing portion can be obtained, that is, the liquid leakage due to the communication of the pores in the in-plane direction of the porous membrane in the channel device can be suppressed, it can be determined that the porous membrane is melted.

For example, in a channel device 103 shown in Fig. 6, the porous membrane 50 is disposed on the first substrate 30 having the groove 31 to form the flow channel 10, and the sealing portion 52 formed by melting at least a portion of the porous membrane 50 in contact with the first substrate 30 is included. Accordingly, it is possible to suppress the liquid leakage due to the communication of the pores of the porous membrane 50 in an in-plane direction. In addition, the second substrate 40 facing the first substrate 30 is disposed on the first substrate 30 through the porous membrane 50.

The channel device may include a plurality of flow channels. Accordingly, cell culture, analysis, and the like can be performed more efficiently. The number and position of the flow channels are not particularly limited and may be appropriately selected depending on the intended purpose.

The channel device may include, for example, a plurality of flow channels in an in-plane direction of the substrate.

In addition, the channel device may include a plurality of flow channels in the thickness direction, for example, by forming a structure in which a plurality of substrates having grooves are laminated through the porous membranes. In such a channel device, at least some of the plurality of flow channels may face each other through the porous membrane.

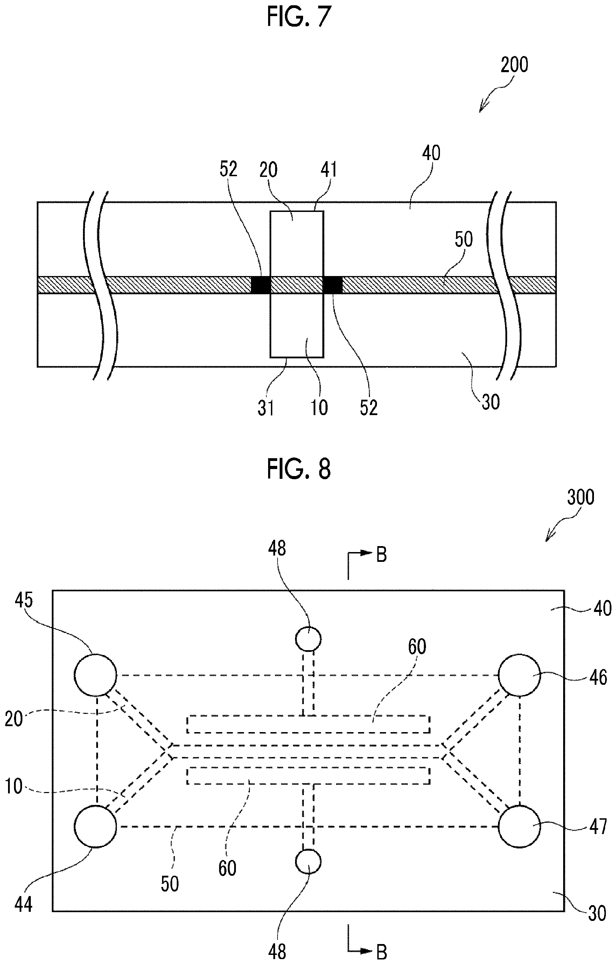

The channel device may include, for example, two flow channels facing each other through the porous membrane. For example, in a channel device 200 shown in Fig. 7, the first substrate 30 having the groove 31 and the second substrate 40 having a groove 41 are laminated via the porous membrane 50. Accordingly, the channel device 200 includes the flow channel 10 and a flow channel 20 facing each other through the porous membrane 50. In addition, at least a portion of the porous membrane 50 in contact with the first substrate 30 and the second substrate 40 is filled with the polymer material and is thus liquid-tightly sealed along the flow channels 10 and 20, whereby the sealing portion 52 is formed. Accordingly, it is possible to suppress the liquid leakage due to the communication of the pores of the porous membrane 50 in an in-plane direction.

[Variable Pressure Chamber]

The channel device may include a variable pressure chamber that is positioned adjacent to the flow channel in a plane direction of the porous membrane and extends along at least a portion of the flow channel. By increasing or decreasing a pressure in the variable pressure chamber, the porous membrane can be deformed in the plane direction. For example, in cell culture applications, by deforming the porous membrane as described above, the channel device can be used in an aspect having a higher biomimetic property.

The channel device may include a plurality of variable pressure chambers, and may include, for example, two variable pressure chambers positioned on both sides of the flow channel.

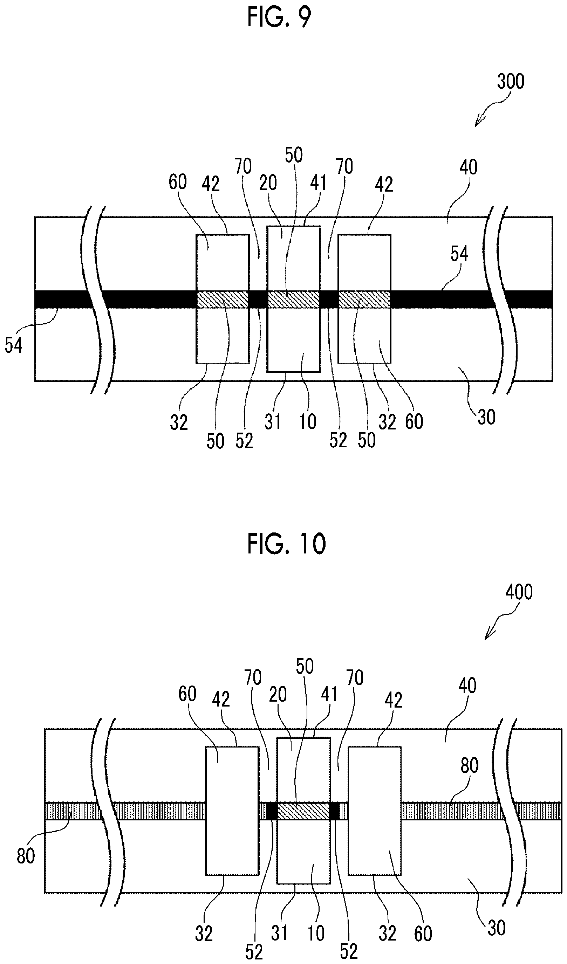

For example, a channel device 300 shown in Fig. 8 and Fig. 9 (cross-sectional view of the channel device 300 of Fig. 8 taken along the line B-B) includes two flow channels and two variable pressure chambers. In the channel device 300, the porous membrane 50 is disposed on the first substrate 30 having the groove 31 to form the flow channel 10. In addition, the second substrate 40 having the groove 41 is disposed on the first substrate 30 to face the groove 31 and forms the second flow channel 20. In addition, at least a portion of the porous membrane 50 in contact with the first substrate 30 and the second substrate 40 is filled with the polymer material and is thus liquid-tightly sealed along the flow channels 10 and 20, whereby the sealing portion 52 is formed. Accordingly, it is possible to suppress the liquid leakage due to the communication of the pores of the porous membrane 50 in an in-plane direction.

In addition, the channel device 300 shown in Figs. 8 and 9 includes two variable pressure chambers 60 that are positioned on both sides of the flow channels 10 and 20 in the plane direction of the porous membrane 50, and extend along at least a portion of the flow channel 10 or 20. The variable pressure chamber 60 is formed of a side groove 32 provided in the first substrate 30 and a side groove 42 provided in the second substrate 40 facing the side groove 32. In addition, in order to secure airtightness of the variable pressure chamber 60, an other region 54 of the porous membrane between the first substrate 30 and the second substrate 40 excluding the sealing portion 52 is filled with the same polymer material as the sealing portion 52 along the variable pressure chamber 60.

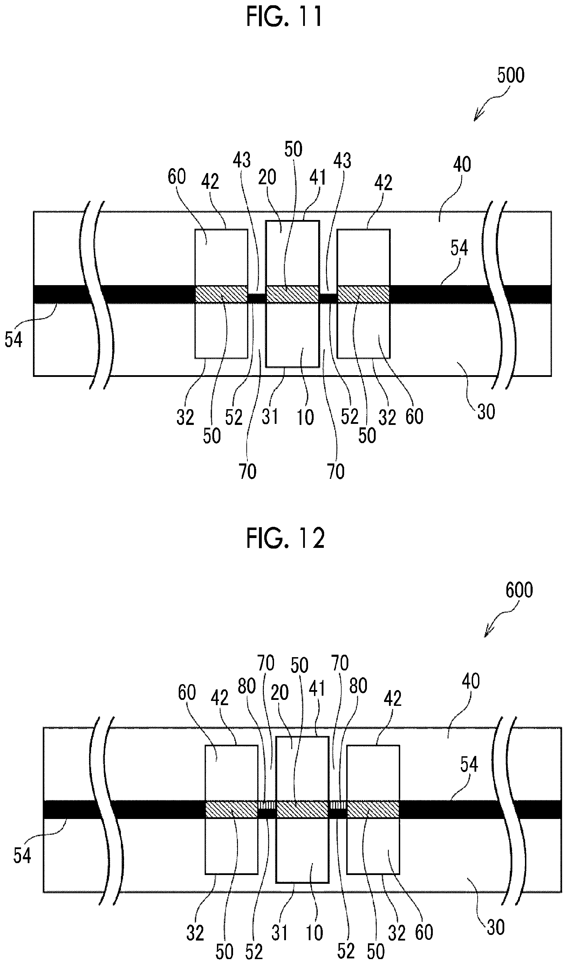

In the channel device 300 shown in Figs. 8 and 9, each of the variable pressure chambers 60 has a structure in which the porous membrane 50 is interposed. However, since the pores of the porous membrane 50 communicate with each other, each of the variable pressure chambers 60 can function as one variable pressure chamber consisting of the side grooves 32 and 42. This also applies to a channel device 500 shown in Fig. 11 and a channel device 600 shown in Fig. 12, which will be described later.

A portion 70 between the flow channels 10 and 20 and the variable pressure chamber 60 (hereinafter, may be referred to as a "flow channel wall") extends in a direction of the variable pressure chamber 60 as the variable pressure chamber 60 is depressurized, and the porous membrane 50 correspondingly extends in the same direction. In addition, as the variable pressure chamber 60 is pressurized, the flow channel wall 70 contracts in a direction of the flow channels 10 and 20, and the porous membrane 50 correspondingly extends in the same direction. In this manner, the porous membrane 50 can be deformed by increasing or decreasing the pressure in the variable pressure chamber 60.

As shown in Fig. 8, the second substrate 40 has a suction port 48 communicating with the variable pressure chamber 60, and the pressure in the variable pressure chamber 60 can be increased or decreased through the suction port 48.

In addition, as shown in Fig. 8, the second substrate 40 has an inlet 44 and an outlet 46 for a sample communicating with the flow channel 10, and an inlet 45 and an outlet 47 communicating with the flow channel 20. Accordingly, the sample can be allowed to flow in the flow channel 10 through the inlet 44 and the outlet 46, and the sample can be allowed to flow in the flow channel 20 through the inlet 45 and the outlet 47. The inlet and the outlet may be reversed.

Examples of another channel device including the two flow channels and the two variable pressure chambers include a channel device 400 shown in Fig. 10. The channel device 400 shown in Fig. 10 is an example including the porous membrane 50 having a width closer to the flow channels 10 and 20 than the channel device 300 shown in Fig. 9. In the channel device 400 shown in Fig. 10, the porous membrane 50 is disposed on the first substrate 30 having the groove 31 to form the flow channel 10. In addition, the second substrate 40 having the groove 41 is disposed on the first substrate 30 to face the groove 31 and forms the second flow channel 20. In addition, at least a portion of the porous membrane 50 in contact with the first substrate 30 and the second substrate 40 is filled with the polymer material and is thus liquid-tightly sealed along the flow channels 10 and 20, whereby the sealing portion 52 is formed. Accordingly, it is possible to suppress the liquid leakage due to the communication of the pores of the porous membrane 50 in an in-plane direction.

In addition, the channel device 400 shown in Fig. 10 includes the two variable pressure chambers 60 that are positioned on both sides of the flow channels 10 and 20 in the plane direction of the porous membrane 50, and extend along at least a portion of the flow channel 10 or 20. The variable pressure chambers 60 are formed of the side grooves 32 provided in the first substrate 30 and the side grooves 42 provided in the second substrate 40 facing the side grooves 32, and are formed on both sides of the flow channels 10 and 20 through the flow channel wall 70. In addition, in order to secure the airtightness of the variable pressure chamber 60, the polymer material 80, which is the same polymer material as the sealing portion 52, is filled between the first substrate 30 and the second substrate 40 along the variable pressure chambers 60 in a region excluding the sealing portion 52.

Examples of another channel device including the two flow channels and the two variable pressure chambers include the channel device 500 shown in Fig. 11. In the channel device 500 shown in Fig. 11, the porous membrane 50 is disposed on the first substrate 30 having the groove 31 to form the flow channel 10. In addition, the second substrate 40 having the groove 41 is disposed on the first substrate 30 to face the groove 31 and forms the second flow channel 20. In addition, at least a portion of the porous membrane 50 in contact with the first substrate 30 and the second substrate 40 is compressed by the protruding portion 43 of the second substrate and is thus liquid-tightly sealed along the flow channels 10 and 20, whereby the sealing portion 52 is formed. Accordingly, it is possible to suppress the liquid leakage due to the communication of the pores of the porous membrane 50 in an in-plane direction.

In addition, the channel device 500 shown in Fig. 11 includes the two variable pressure chambers 60 that are positioned on both sides of the flow channels 10 and 20 in the plane direction of the porous membrane 50, and extend along at least a portion of the flow channel 10 or 20. The variable pressure chambers 60 are formed of the side grooves 32 provided in the first substrate 30 and the side grooves 42 provided in the second substrate 40 facing the side grooves 32, and are formed on both sides of the flow channels 10 and 20 through the flow channel wall 70. In addition, in order to secure the airtightness of the variable pressure chamber 60, the other region 54 of the porous membrane between the first substrate 30 and the second substrate 40 is also filled with the polymer material along the variable pressure chamber 60.

Examples of another channel device including the two flow channels and the two variable pressure chambers include the channel device 600 shown in Fig. 12. In the channel device 600 shown in Fig. 12, the porous membrane 50 is disposed on the first substrate 30 having the groove 31 to form the flow channel 10. In addition, the second substrate 40 having the groove 41 is disposed on the first substrate 30 to face the groove 31 and forms the second flow channel 20. In addition, at least a portion of the porous membrane 50 in contact with the first substrate 30 and the second substrate 40 is melted and is thus liquid-tightly sealed along the flow channels 10 and 20, whereby the sealing portion 52 is formed. Accordingly, it is possible to suppress the liquid leakage due to the communication of the pores of the porous membrane 50 in an in-plane direction.

In addition, the channel device 600 shown in Fig. 12 includes the two variable pressure chambers 60 that are positioned on both sides of the flow channels 10 and 20 in the plane direction of the porous membrane 50, and extend along at least a portion of the flow channel 10 or 20. The variable pressure chambers 60 are formed of the side grooves 32 provided in the first substrate 30 and the side grooves 42 provided in the second substrate 40 facing the side grooves 32, and are formed on both sides of the flow channels 10 and 20 through the flow channel wall 70. In addition, in order to secure the air-tightness of the variable pressure chamber 60, the other region 54 of the porous membrane between the first substrate 30 and the second substrate 40 is also filled with the polymer material along the variable pressure chamber 60, and furthermore, a portion on the sealing portion 52 is also filled with the polymer material 80, which is the same polymer material as the region 54.

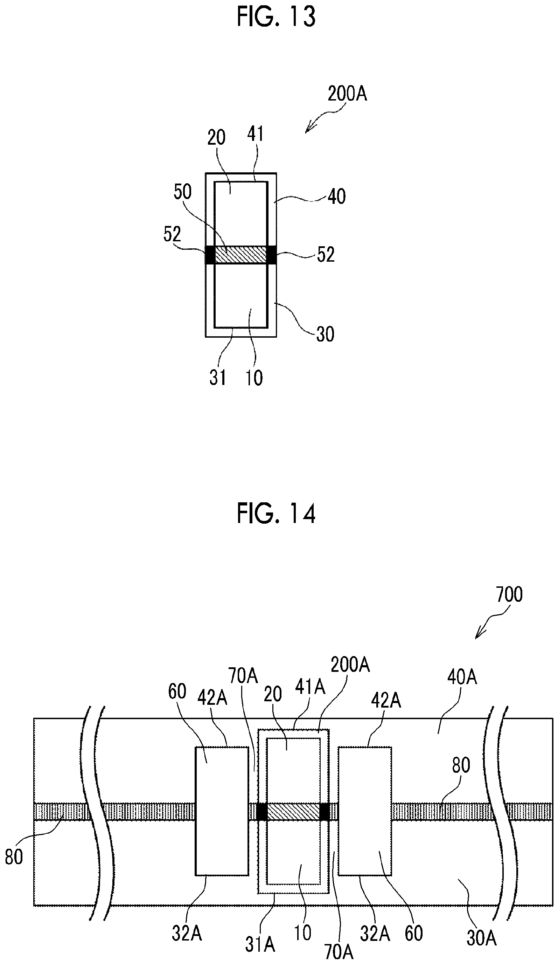

Examples of another channel device including the two flow channels and the two variable pressure chambers include a channel device 700 shown in Fig. 14. In the channel device 700 shown in Fig. 14, a channel device 200A shown in Fig. 13, which is a unit having a function of a flow channel, is assembled into a unit having a function of a variable pressure chamber.

In the channel device 200A, the porous membrane 50 is disposed on the first substrate 30 having the groove 31 to form the flow channel 10. In addition, the second substrate 40 having the groove 41 is disposed on the first substrate 30 to face the groove 31 and forms the second flow channel 20. In addition, at least a portion of the porous membrane 50 in contact with the first substrate 30 and the second substrate 40 is filled with the polymer and is thus liquid-tightly sealed along the flow channels 10 and 20, whereby the sealing portion 52 is formed. Accordingly, it is possible to suppress the liquid leakage due to the communication of the pores of the porous membrane 50 in an in-plane direction.

Further, in the channel device 700 shown in Fig. 14, a channel device 200A is accommodated between a groove 31A provided in another first substrate 30A and a groove 41A provided in another second substrate 40A. Accordingly, the channel device 400 includes the flow channels 10 and 20 formed of the grooves 31A and 42A and the porous membrane 50. In addition, the channel device 700 includes the two variable pressure chambers 60 that are positioned on both sides of the flow channels 10 and 20 in the plane direction of the porous membrane 50, and extend along at least a portion of the flow channel 10 or 20. The variable pressure chamber 60 is formed of a side groove 32A provided in the first substrate 30A and a side groove 42A provided in the second substrate 40A facing the side groove 32A. In addition to the sealing portion 52, the polymer material 80, which is the same polymer material as the sealing portion 52, is also filled between the first substrate 30A and the second substrate 40A and sealed along the variable pressure chamber 60.

A portion 70A between the flow channels 10 and 20 and the variable pressure chamber 60, that is, a flow channel wall 70A can also be deformed by increasing or decreasing the pressure in the variable pressure chamber 60, and the porous membrane 50 can be correspondingly deformed.

[Other Materials]

In addition to the above, the channel device may use an adhesive material for adhesion between the substrate and the porous membrane or between the substrate and the substrate. As the adhesive material, for example, the above-described polymer material can be used.

Portions of the porous membrane other than the portions where the flow channel and the sealing portion are formed may be filled with the adhesive material, for example, the polymer material. The same applies to the exemplary drawings of the present disclosure.

In the exemplary drawings of the present disclosure, the adhesive material may be omitted for the sake of simplicity.

<Manufacturing Method of Channel Device>

The manufacturing method of the channel device according to the embodiment of the present disclosure is not particularly limited, but the channel device can be preferably manufactured by the manufacturing method of the channel device according to the embodiment of the present disclosure described below.

The manufacturing method of the channel device according to the embodiment of the present disclosure includes
a step of disposing a porous membrane having pores communicating in a direction intersecting a thickness direction thereof on a first substrate having a first groove to form a first flow channel formed of the first groove and the porous membrane, and liquid-tightly sealing at least a portion of the porous membrane in contact with the first substrate along the first flow channel.

[Substrate]

As the substrate, those described above can be used.

A manufacturing method of the substrate is not particularly limited, and a known method may be used. In a case where the substrate is made of a resin, a substrate having a desired groove can be easily obtained by pouring a resin material onto a forming die. Such a forming die may be prepared by using a known method. For example, a forming die may be prepared by performing a cutting process on a resin plate to form a protruding portion having an inverted shape of a groove or the like, and a resin material may be poured onto the obtained forming die and cured to obtain a substrate having a groove.

[Porous Membrane]

As the porous membrane, those described above can be used.

[Sealing]

A sealing portion is formed by disposing the porous membrane on the first substrate having the first groove to form the first flow channel formed of the first groove and the porous membrane, and liquid-tightly sealing at least a portion of the porous membrane in contact with the first substrate along the first flow channel.

A method of forming the sealing portion is not particularly limited as long as the porous membrane is liquid-tightly sealed along the flow channel, and may be appropriately selected in consideration of the application of the channel device and the like. As the method of forming the sealing portion, as described below, (1) a method of filling the porous membrane with a polymer material, (2) a method of compressing the porous membrane, and (3) a method of melting the porous membrane are preferable.

-Method of Forming Sealing Portion by Filling Porous Membrane with Polymer Material-

Sealing may be performed by filling at least a portion of the porous membrane in contact with the substrate with the polymer material. The fact that the porous membrane is "filled" with the polymer material and a filling ratio of the polymer material are as described above.

The polymer material may be a precursor monomer of the above-described polymer and the resin. In the present disclosure, the polymer material before and after curing is not distinguished and may be referred to as "polymer material".

In addition, the polymer material preferably contains a curing agent component in addition to the above-described components. A type of the curing agent may be appropriately selected depending on the type of the polymer material, and examples thereof include a photocuring agent such as a photocationic curing initiator and a photoradical polymerization curing agent, and a thermosetting agent such as an azo-based polymerization initiator and an organic peroxide-based polymerization initiator.

One type of the curing agent may be used alone, or two or more types thereof may be used in combination.

In a case where the polymer material contains the curing agent, the amount of the curing agent is, for example, 1 mass% to 10 mass% with respect to the total solid content amount of the polymer material, although the amount of the curing agent depends on the type thereof.

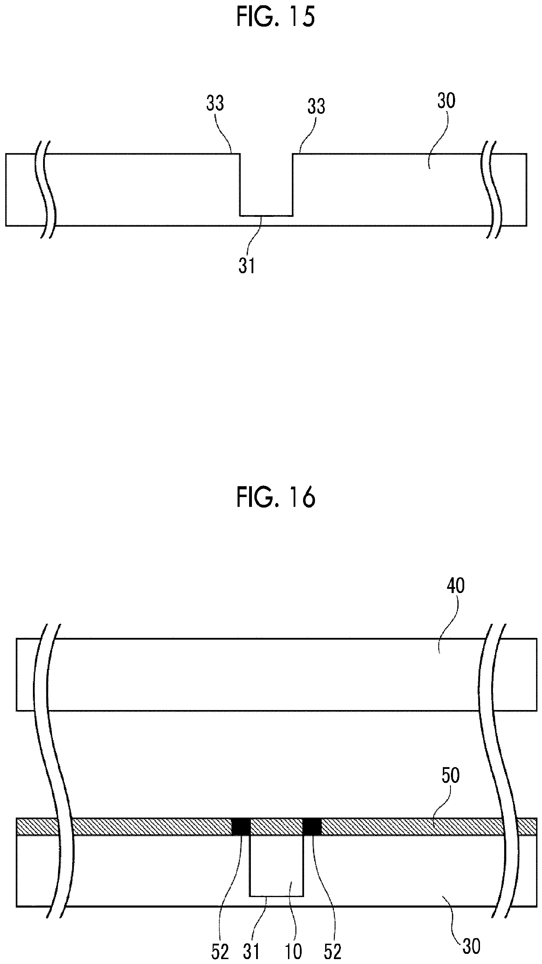

For example, as shown in Fig. 15, the first substrate 30 having the first groove 31 is prepared. For example, after applying the polymer material to a region 33 on the first substrate 30, the porous membrane may be disposed on the first substrate 30. Alternatively, for example, after disposing the porous membrane on the first substrate 30, the polymer material may be applied to a portion of the porous membrane in contact with the region 33 on the first substrate 30.

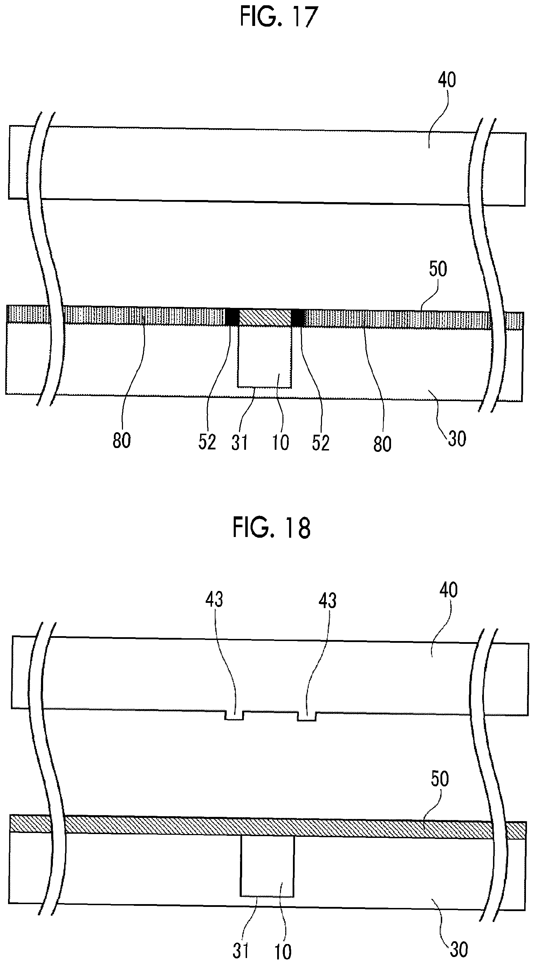

Accordingly, for example, as shown in Fig. 16 or 17, the first flow channel 10 formed of the first groove 31 and the porous membrane 50 can be formed, and the sealing portion 52 can be formed by liquid-tightly sealing at least a portion of the porous membrane 50 in contact with the first substrate 30 along the first flow channel 10.

A step shown in Fig. 17 is an example in which the porous membrane 50 having a width closer to that of the first flow channel 10 is used compared to a step shown in Fig. 16. In addition, in the step shown in Fig. 17, the polymer material 80 is applied to portions of the first substrate 30 excluding the sealing portion 52.

In a case where a layer of an extracellular matrix is formed on a surface of the porous membrane, from the viewpoint of more easily filling the porous membrane 50 with the polymer material, the layer of the extracellular matrix may be small in thickness, and is preferably, for example, about 1 nm to 1 $\mu$m in thickness.

In addition, in a case where the layer of the extracellular matrix is formed on the surface of the porous membrane, from the viewpoint of more easily filling the porous membrane 50 with the polymer material, the layer of the extracellular matrix may be peeled off from a portion of the porous membrane to which the polymer material is to be applied.

A method of applying the polymer material is not particularly limited. For example, after applying the polymer material to a plate such as a glass plate by a spin coating method, a bar coater method, or the like, the polymer material on the plate may be transferred onto the first substrate or the porous membrane to be applied thereto.

After forming the sealing portion 52, for example, as shown in Fig. 16, the second substrate 40 may be disposed on the first substrate 30 from above the porous membrane 50 through the porous membrane 50. Accordingly, the channel device 100 shown in Fig. 2 can be obtained.

In addition, for example, as shown in Fig. 17, the second substrate 40 may be disposed on the first substrate 30 through the porous membrane 50 from above the porous membrane 50. Accordingly, the channel device 101 shown in Fig. 4 can be obtained.

-Method of Forming Sealing Portion by Compressing Porous Membrane-

Sealing may be performed by compressing at least a portion of the porous membrane in contact with the substrate. The fact that the porous membrane is "compressed" is as described above.

A method of compressing the porous membrane is not particularly limited, and examples thereof include a method of bending the porous membrane and a method of pressing the porous membrane.

For example, as shown in Fig. 15, the first substrate 30 having the first groove 31 is prepared. For example, as shown in Fig. 18, by disposing the porous membrane 50 on the first substrate 30, the first flow channel 10 formed of the first groove 31 and the porous membrane 50 can be formed. In addition, as shown in Fig. 18, by disposing the second substrate 40 on the first substrate 30 through the porous membrane 50 from above the porous membrane 50, the protruding portion 43 of the second substrate 40 presses the porous membrane. Accordingly, at least a portion of the porous membrane 50 in contact with the first substrate 30 can be liquid-tightly sealed along the first flow channel 10 and the sealing portion 52 can be formed, so that the channel device 102 shown in Fig. 5 can be obtained.

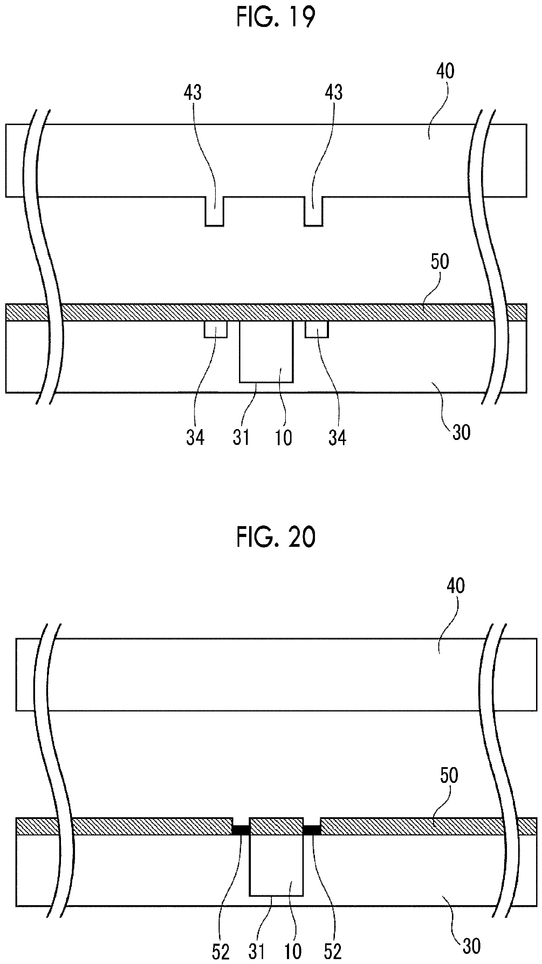

From the viewpoint of preventing the formation of a gap due to a thickness of the protruding portion 43 between the first substrate 30 and the second substrate 40 and the porous membrane 50, the thickness of the protruding portion 43 is preferably equal to or less than the thickness of the porous membrane 50. Alternatively, from the same viewpoint, for example, as shown in Fig. 19, the first substrate 30 may have a recessed portion 34 into which the protruding portion 43 is fitted.

-Method of Forming Sealing Portion by Melting Porous Membrane-

Sealing may be performed by melting at least a portion of the porous membrane in contact with the substrate. The fact that the porous membrane is "melted" is as described above.

A method of melting the porous membrane is not particularly limited, and examples thereof include a method of pressing a jig heated to a temperature equal to or higher than a melting point of the porous membrane and equal to or lower than a melting point of the first substrate against the porous membrane, and a laser irradiation method.

For example, as shown in Fig. 15, the first substrate 30 having the first groove 31 is prepared.

In a case where the porous membrane is melted by the method of pressing a heated jig against the porous membrane, for example, after disposing the porous membrane on the first substrate 30, a portion of the porous membrane in contact with the region 33 on the first substrate 30 is pressed by a linear jig heated to a temperature equal to or higher than the melting point of the porous membrane and equal to or lower than the melting point of the first substrate 30 to melt the porous membrane.

In addition, in a case where the porous membrane is melted by the laser irradiation method, for example, after disposing a porous membrane made of a material that absorbs laser light on the first substrate 30, a portion of the porous membrane in contact with the region 33 on the first substrate 30 is irradiated with laser light. Light energy absorbed by the porous membrane is converted into heat to melt the porous membrane.

In the manner described above, for example, as shown in Fig. 20, the first flow channel 10 formed of the first groove 31 and the porous membrane 50 can be formed, and the sealing portion 52 can be formed by liquid-tightly sealing at least a portion of the porous membrane 50 in contact with the first substrate 30 along the first flow channel 10.

After forming the sealing portion 52, for example, as shown in Fig. 20, the second substrate 40 may be disposed on the first substrate 30 through the porous membrane 50 from above the porous membrane 50. Accordingly, the channel device 103 shown in Fig. 6 can be obtained.

In addition, in the manufacturing method of the channel device according to the embodiment of the present disclosure,

the first substrate may have two first side grooves positioned on both sides of the first groove and provided along at least a portion of the first groove, and the manufacturing method of the channel device according to the embodiment of the present disclosure may include a step of disposing a second substrate having a second groove and two second side grooves on the first substrate to respectively face the first groove and the two first side grooves through the porous membrane and forms a second flow channel formed of the second groove and the porous membrane and two variable pressure chambers formed of the first side grooves and the second side grooves.

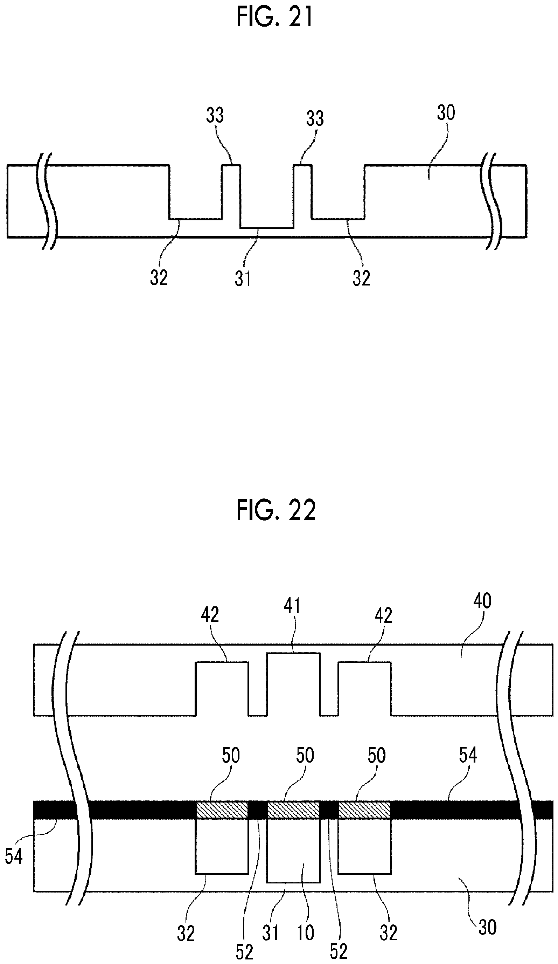

For example, as shown in Fig. 21, the first substrate 30 having the first groove 31 and the two first side grooves 32 that are positioned on both sides of the first groove 31 and are provided along at least a portion of the first groove 31 is prepared.

In a case where sealing is performed by filling the porous membrane with the polymer material, after applying the polymer material to the region 33 on the first substrate 30, the porous membrane may be disposed on the first substrate 30. Alternatively, for example, after disposing the porous membrane on the first substrate 30, the polymer material may be applied to a portion of the porous membrane in contact with the region 33 on the first substrate 30.

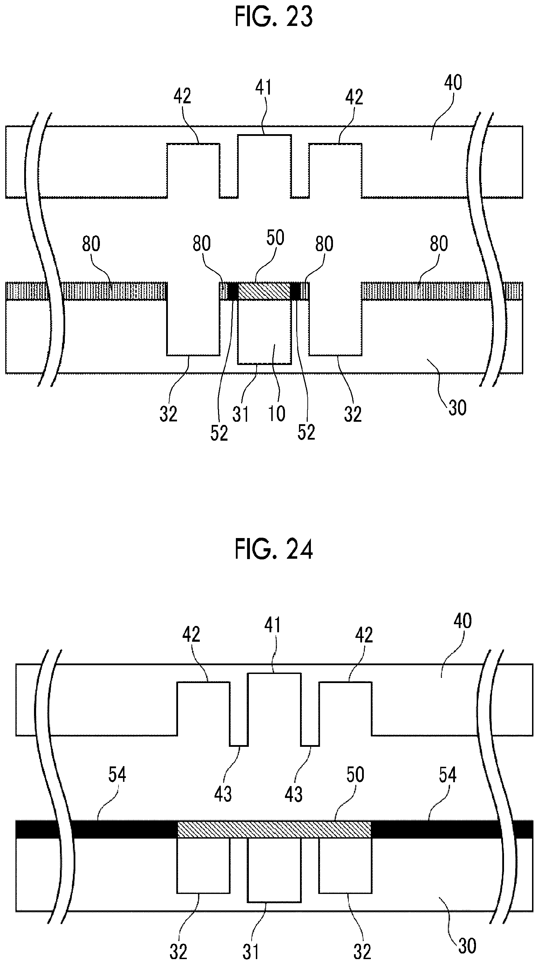

Accordingly, for example, as shown in Fig. 22 or 23, the first flow channel 10 formed of the first groove 31 and the porous membrane 50 can be formed, and the sealing portion 52 can be formed by liquid-tightly sealing at least a portion of the porous membrane 50 in contact with the first substrate 30 along the first flow channel 10.

In a step shown in Fig. 22, in order to secure air-tightness of the variable pressure chambers to be formed later, the same polymer material as the sealing portion 52 is also applied to the other region 54 of the porous membrane on the first substrate 30 excluding the sealing portion 52.

A step shown in Fig. 23 is an example in which the porous membrane 50 having a width closer to that of the first flow channel 10 is used compared to a step shown in Fig. 22. In addition, in the step shown in Fig. 23, in order to secure the air-tightness of the variable pressure chambers to be formed later, the polymer material 80 is applied to portions of the first substrate 30 excluding the sealing portion 52.

For example, as shown in Figs. 22 and 23, the second substrate 40 having the second groove 41 and the two second side grooves 42 that are positioned on both sides of the second groove 41 and are provided along at least a portion of the second groove 41 is prepared.

After forming the sealing portion 52, as shown in Fig. 22, the second substrate is disposed on the first substrate from above the porous membrane 50 so that the second groove 41 and the two second side grooves 42 respectively face the first groove 31 and the two first side grooves 32 through the porous membrane 50, whereby the second flow channel 20 formed of the second groove 41 and the porous membrane 50 and the two variable pressure chambers formed of the first side grooves 32 and the second side grooves 42 are formed. In addition, at least a portion of the porous membrane 50 in contact with the second substrate 40 is liquid-tightly sealed along the second flow channel 20 by the sealing portion 52. In this manner, the channel device 300 shown in Fig. 9 can be obtained.

In addition, after forming the sealing portion 52, as shown in Fig. 23, in the same manner as in the step shown in Fig. 22, the second flow channel 20 formed of the second groove 41 and the porous membrane 50, and the two variable pressure chambers formed of the first side grooves 32 and the second side grooves 42 are formed. In addition, at least a portion of the porous membrane 50 in contact with the second substrate 40 is liquid-tightly sealed along the second flow channel 20 by the sealing portion 52. In this manner, the channel device 400 shown in Fig. 10 can be obtained.

In a case where sealing is performed by compressing the porous membrane, as shown in Fig. 24, by disposing the porous membrane 50 on the first substrate 30, the first flow channel 10 formed of the first groove 31 and the porous membrane 50 can be formed. In addition, in order to secure the air-tightness of the variable pressure chambers to be formed later, the same polymer material is also applied to the other region 54 of the porous membrane on the first substrate 30.

As shown in Fig. 24, the second substrate is disposed on the first substrate from above the porous membrane 50 so that the second groove 41 and the two second side grooves 42 respectively face the first groove 31 and the two first side grooves 32 through the porous membrane 50, whereby the second flow channel 20 formed of the second groove 41 and the porous membrane 50 and the two variable pressure chambers formed of the first side grooves 32 and the second side grooves 42 are formed. At that time, the porous membrane 50 is pressed by the protruding portion 43 of the second substrate 40. Accordingly, the sealing portion 52 can be formed by liquid-tightly sealing at least a portion of the porous membrane 50 in contact with the first substrate 30 along the first flow channel 10 and the second flow channel 20. At the same time, at least a portion of the porous membrane 50 in contact with the second substrate 40 is liquid-tightly sealed along the second flow channel 20 by the sealing portion 52. In this manner, the channel device 500 shown in Fig. 11 can be obtained.

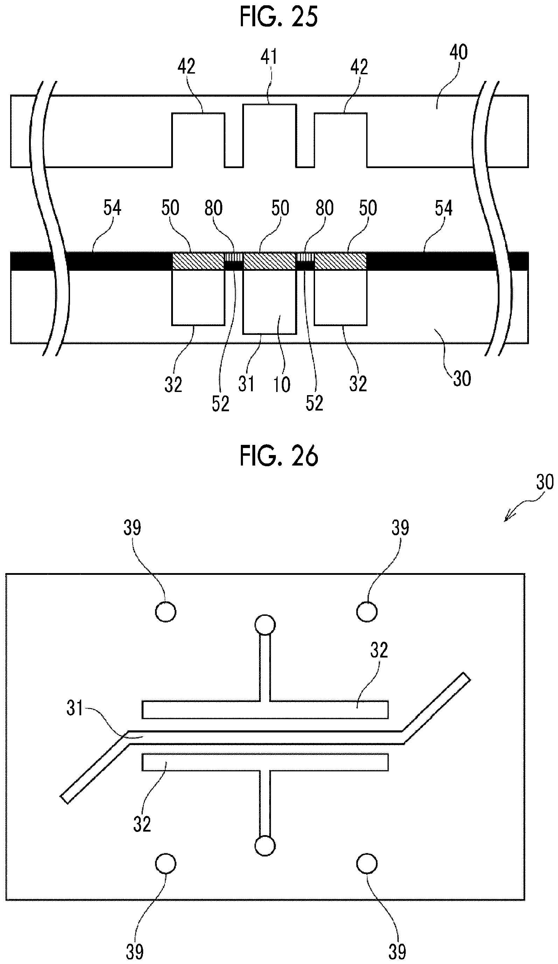

In a case where sealing is performed by melting the porous membrane, as shown in Fig. 25, after disposing the porous membrane 50 on the first substrate 30, the porous membrane is melted by the method of pressing a linear jig heated to a temperature equal to or higher than the melting point of the porous membrane and equal to or lower than the melting point of the first substrate 30 against a portion of the porous membrane in contact with the region 33 on the first substrate 30, the laser irradiation method, or the like.

Accordingly, as shown in Fig. 25, the first flow channel 10 formed of the first groove 31 and the porous membrane 50 can be formed, and the sealing portion 52 can be formed by liquid-tightly sealing at least a portion of the porous membrane 50 in contact with the first substrate 30 along the flow channel 10. In addition, in order to secure the air-tightness of the variable pressure chamber to be formed later, the polymer material is also applied to the other region 54 of the porous membrane on the first substrate 30, and furthermore, the polymer material 80, which is the same polymer material as the region 54, is also applied onto the sealing portion 52.

After forming the sealing portion 52, for example, as shown in Fig. 25, the second substrate is disposed on the first substrate from above the porous membrane 50 so that the second groove 41 and the two second side grooves 42 respectively face the first groove 31 and the two first side grooves 32 through the porous membrane 50, whereby the second flow channel 20 formed of the second groove 41 and the porous membrane 50 and the two variable pressure chambers formed of the first side grooves 32 and the second side grooves 42 are formed. In addition, at least a portion of the porous membrane 50 in contact with the second substrate 40 is liquid-tightly sealed along the second flow channel 20 by the sealing portion 52. In this manner, the channel device 600 shown in Fig. 12 can be obtained.

[Other Steps]

The manufacturing method of the channel device according to the embodiment of the present disclosure may include a step of applying an adhesive material to a lower surface of the second substrate 40 before disposing the second substrate 40 on the first substrate 30 through the porous membrane 50. As the adhesive material, the above-described polymer material may be used. A method of applying the adhesive material may be the same as the method described above for the polymer material.

In addition, in order to accelerate the curing of the polymer material, a heating step may be appropriately included. Conditions such as a heating temperature and a heating time may be appropriately adjusted in consideration of the type of the polymer material and the like.

Examples

Hereinafter, the present disclosure will be described in more detail with reference to Examples. However, the present disclosure is not limited to these Examples.

[Substrate]

In order to produce the first substrate 30 shown in Fig. 26, a forming die for a first substrate was prepared by performing a cutting process on an acrylic plate to form a protruding portion having an inverted shape of a groove or the like. Polydimethylsiloxane ("SYLGARD 184" manufactured by Dow (a two-part type consisting of a base agent and a curing agent)) was adjusted so that a mass ratio between the base agent and the curing agent was 10:1, and was mixed with a vacuum centrifuge. The mixed polydimethylsiloxane was poured onto the forming die for a first substrate, allowed to stand under reduced pressure for 3 hours to defoam, and then heated in a constant temperature chamber at 50°C for 15 hours to cure. In this manner, the first substrate 30 having the first groove 31 and the two first side grooves 32 that were positioned on both sides of the first groove 31 and were provided along at least a portion of the first groove 31 was produced.

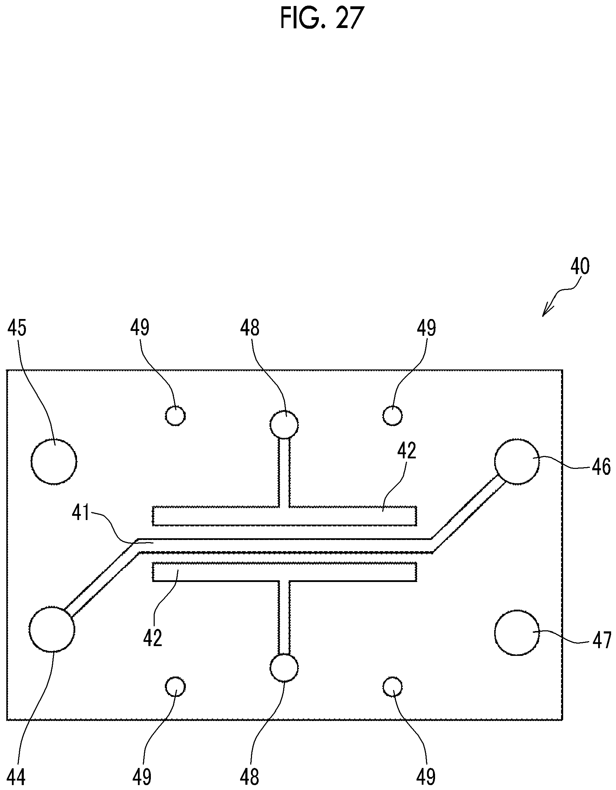

Similarly, as shown in Fig. 27, the second substrate 40 having the second groove 41 and the two second side grooves 42 that were positioned on both sides of the second groove 41 and were provided along at least a portion of the second groove 41 was produced. In addition, the second substrate 40 had the inlet 44 and the outlet 46, and the inlet 45 and the outlet 47.

Both the first substrate 30 and the second substrate 40 had dimensions of 40 mm $\times$ 25 mm $\times$ 3 mm. In addition, for convenience in combining the first substrate 30 and the second substrate 40, the first substrate 30 had four positioning holes 39, and the second substrate 40 had four positioning holes 49.

[Porous Membrane]

A honeycomb film made of polybutadiene was used as the porous membrane 50. The honeycomb film was produced based on the description of JP4904025B.

The honeycomb film was observed using a microscope "VHX-7000" manufactured by Keyence Corporation. A surface of the honeycomb film had through-holes in which centers of pores were regularly arranged in units of hexagonal parallelogons. In a case where each through-hole was viewed from a cross-sectional direction of the film, the through-hole had a spherical segment shape (a shape in which an upper end and a lower end of a sphere were cut), and the through-holes adjacent to each other communicated with each other through communication pores inside the porous membrane.

In a case where an average opening diameter and an opening ratio of the honeycomb film were measured as follows, the average opening diameter of the through-holes was about 5 μm, and the opening ratio thereof was about 60%. An average distance between the through-holes was about 6 μm.

• Measurement of Average Opening Diameter

Any 300 μm square range of the surface of the honeycomb film was imaged with a microscope, and an area of each of all the pores within the range was calculated from the number of pixels imaged. A diameter of a circle corresponding to the pore area was defined as an opening diameter, and an arithmetic average value thereof was defined as the average opening diameter.

· Measurement of Opening Ratio

A surface of the porous membrane was imaged with a microscope, and areas (S1) of openings were calculated from the number of pixels imaged. Similarly, an area (S2) of the porous membrane including the openings was calculated. An opening ratio (P) was obtained by the following formula.

$$P = S1 / S2 \times 100$$

[Production of Channel Device]

In the following procedure, the channel device 100 of Examples as shown in Figs. 8 and 9 was produced using the first substrate 30 shown in Fig. 26 and the second substrate 40 shown in Fig. 27.

· A flat surface seat having four pins for positioning were prepared at a predetermined position.
· Polydimethylsiloxane used in the production of the first substrate 30 and the second substrate 40 was used as the polymer material. Polydimethylsiloxane was applied onto a glass plate to form a polydimethylsiloxane coating film having a thickness of 5 μm. The polydimethylsiloxane coating film was transferred onto the entire upper surface (excluding the groove 31 and the side grooves 32) of the first substrate 30 as shown in Fig. 21 to apply the polydimethylsiloxane.
· The first substrate 30 was installed on the flat surface seat while passing the four pins of the flat surface seat through the positioning holes 39 of the first substrate 30.
· The porous membrane 50 was cut into a size capable of covering the first groove 31, the second groove 42, the side grooves 32, and the side grooves 42, and the porous membrane 50 was disposed on the first substrate 30. In this manner, as shown in Fig. 22, the first flow channel 10 formed of the first groove 31 and the porous membrane 50, at least a portion of the porous membrane 50 in contact with the first substrate 30 was filled with polydimethylsiloxane and was thus liquid-tightly sealed along the first flow channel 10, whereby the sealing portion 52 was formed. At the same time, as shown in Fig. 22, the polymer material was also applied to the other region 54 of the porous membrane on the first substrate 30.
· Polydimethylsiloxane was also applied to the entire lower surface of the second substrate 40 (excluding the groove 41 and the side grooves 42) as shown in Fig. 22 in the same manner as in the first substrate 30.
· While inserting the four pins of the flat surface seat through the positioning holes 49 of the second substrate 40, as shown in Fig. 22, the second substrate was disposed on the first substrate from above the porous membrane 50 so that the second groove 41 and the two second side grooves 42 respectively faced the first groove 31 and the two first side grooves 32 through the porous membrane 50. Accordingly, the second flow channel 20 formed of the second groove 41 and the porous membrane 50, and the two variable pressure chambers 60 formed of the first side grooves 32 and the second side grooves 42 were formed. In addition, at least a portion of the porous membrane 50 in contact with the second substrate 40 was liquid-tightly sealed along the second flow channel 20 by the sealing portion 52.
• After allowing the resultant to stand at room temperature under normal pressure for 2 hours, the resultant was heated in a constant temperature chamber at 50°C for 3 hours.

[Evaluation]

A red liquid (a mixture of 99.9 mass% of ethanol having a concentration of 70 mass% and 0.1 mass% of a red food dye) and a green liquid (a mixture of 99.9 mass% of ethanol having a concentration of 70 mass% and 0.1 mass% of a green food dye) were prepared.
After injecting 100 μl of the red liquid into the first flow channel 10 and injecting 100 μl of the green liquid into the second flow channel 20, the channel device 100 was placed in a container, sealed, and left for one day. As a result of observing the channel device 100 after being left for one day, it was confirmed that there was no liquid leakage due to the communication of the pores in the in-plane direction of the porous membrane, and the first flow channel 10 and the second flow channel 20 were liquid-tightly sealed.
The disclosure of JP2021-013761 filed on January 29, 2021 is incorporated herein by reference in its entirety. All references, patent applications, and technical standards described herein are incorporated herein by reference to the same extent as if the individual references, patent applications, and technical standards were specifically and individually stated to be incorporated by reference.

**Claims**

1. A channel device comprising:

   a flow channel formed of a groove provided in a substrate and a porous membrane having pores communicating in a direction intersecting a thickness direction of the porous membrane, wherein at least a portion of the porous membrane in contact with the substrate is liquid-tightly sealed along the flow channel.

2. The channel device according to claim 1, further comprising:
   a plurality of the flow channels.

3. The channel device according to claim 2, wherein the channel device includes two flow channels facing each other through the porous membrane.

4. The channel device according to any one of claims 1 to 3, further comprising:
   a variable pressure chamber that is positioned adjacent to the flow channel in a plane direction of the porous membrane and extends along at least a portion of the flow channel.

5. The channel device according to claim 4, further comprising:
   the two variable pressure chambers positioned on both sides of the flow channel.

6. The channel device according to any one of claims 1 to 5,
   wherein at least the portion of the porous membrane in contact with the substrate is filled with a polymer material.

7. The channel device according to any one of claims 1 to 5,
   wherein at least the portion of the porous membrane in contact with the substrate is compressed.

8. The channel device according to any one of claims 1 to 5,
   wherein at least the portion of the porous membrane in contact with the substrate is melted.

9. The channel device according to any one of claims 1 to 8,
   wherein the porous membrane has an opening ratio of 10% to 70% and an average opening diameter of 0.01 $\mu$m to 100 $\mu$m.

10. The channel device according to any one of claims 1 to 9,
    wherein a layer of an extracellular matrix is formed on a surface of a portion of the porous membrane that forms the flow channel.

11. The channel device according to claim 10,
    wherein the extracellular matrix is not provided in the groove.

12. A manufacturing method of a channel device, comprising:
    a step of disposing a porous membrane having pores communicating in a direction intersecting a thickness direction of the porous membrane on a first substrate having a first groove to form a first flow channel formed of the first groove and the porous membrane, and liquid-tightly sealing at least a portion of the porous membrane in contact with the first substrate along the first flow channel.

13. The manufacturing method of a channel device according to claim 12,
    wherein the sealing is performed by filling at least the portion of the porous membrane in contact with the first substrate with a polymer material.

14. The manufacturing method of a channel device according to claim 12,
    wherein the sealing is performed by compressing at least the portion of the porous membrane in contact with the first substrate.

15. The manufacturing method of a channel device according to claim 12,
    wherein the sealing is performed by melting at least the portion of the porous membrane in contact with the first substrate.

16. The manufacturing method of a channel device according to any one of claims 12 to 15, in which the first substrate has two first side grooves positioned on both sides of the first groove and provided along at least a portion of the first groove, the manufacturing method further comprising:
    a step of disposing a second substrate having a second groove and two second side grooves on the first substrate to cause the second groove and the two second side grooves to respectively face the first groove and the two first side grooves through the porous membrane to form a second flow channel formed of the second groove and the porous membrane and two variable pressure chambers formed of the first side grooves and the second side grooves.

FIG. 1

FIG. 2

# FIG. 3

# FIG. 4

# FIG. 5

102

40

43    43    50

52  31  10  52    30

# FIG. 6

103

40

50

52  31  10  52    30

# FIG. 7

# FIG. 8

## FIG. 9

## FIG. 10

## FIG. 11

## FIG. 12

## FIG. 13

## FIG. 14

# FIG. 15

# FIG. 16

## FIG. 17

## FIG. 18

## FIG. 19

## FIG. 20

## FIG. 21

## FIG. 22

EP 4 269 552 A1

## FIG. 23

## FIG. 24

30

# FIG. 25

# FIG. 26

# FIG. 27

## INTERNATIONAL SEARCH REPORT

| International application No. |
|---|
| **PCT/JP2022/003394** |

**A. CLASSIFICATION OF SUBJECT MATTER**

***C12M 1/00***(2006.01)i; ***G01N 37/00***(2006.01)i
FI: C12M1/00 D; G01N37/00 101

According to International Patent Classification (IPC) or to both national classification and IPC

**B. FIELDS SEARCHED**

Minimum documentation searched (classification system followed by classification symbols)

C12M1/00; G01N37/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WANG, Z. F., SEAH, Y. P., WANG, Z. P. Seamless joining of porous membrane with thermoplastic microfluidic devices. Microelectronic Engineering. 2013, vol. 110, pages 386-391, http://dx.doi.org/10.1016/j.mee.2013.02.074 particularly, summary, fig. 2, 4 | 1-3, 6-12, 14-15 |
| Y | particularly, summary, fig. 2, 4 | 1-16 |
| X | CHUEH, Bor-han et al. Leakage-Free Bonding of Porous Membranes into Layered Microfluidic Array Systems. Anal. Chem. 2007, vol. 79, pages 3504-3508 particularly, summary, fig. 2 | 1-3, 6, 9-15 |
| Y | particularly, summary, fig. 2 | 1-16 |
| Y | JP 2017-504320 A (PRES. AND FELL. OF HARVARD COLLEGE) 09 February 2017 (2017-02-09) claims 1, 18-19, fig. 24, description thereof, fig. 2D | 1-16 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 April 2022** | **12 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/003394**

| Patent document cited in search report | Publication date (day/month/year) | Patent family member(s) | Publication date (day/month/year) |
|---|---|---|---|
| JP 2017-504320 A | 09 February 2017 | US 2016/0313306 A1<br>claims 1, 18-19, fig. 2A, 2B, 2D | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016000051 A
- JP 2017136082 A
- JP 4734157 B
- JP 4904025 B
- JP 4945281 B
- JP 5405374 B
- JP 5422230 B
- JP 2011074140 A
- JP 2021013761 A